(19) **Europäisches Patentamt**

**European Patent Office**

**Office européen des brevets**

(11) Veröffentlichungsnummer: **0 364 613 B1**

(12) **EUROPÄISCHE PATENTSCHRIFT**

(45) Veröffentlichungstag der Patentschrift: **29.12.93**

(51) Int. Cl.⁵: **A61B 6/03**

(21) Anmeldenummer: **88117262.1**

(22) Anmeldetag: **17.10.88**

(54) **Verfahrenzum Betrieb eines Computertomographen.**

(43) Veröffentlichungstag der Anmeldung:
**25.04.90 Patentblatt 90/17**

(45) Bekanntmachung des Hinweises auf die
Patenterteilung:
**29.12.93 Patentblatt 93/52**

(84) Benannte Vertragsstaaten:
**DE FR GB**

(56) Entgegenhaltungen:
**DE-A- 2 642 846**
**FR-A- 2 249 517**
**FR-A- 2 387 634**
**GB-A- 2 005 953**

(73) Patentinhaber: **SIEMENS AKTIENGESELL-**
**SCHAFT**
**Wittelsbacherplatz 2**
**D-80333 München(DE)**

(72) Erfinder: **Klingenbeck, Klaus, Dr.**
**Dannberger Weg 8**
**D-8521 Niederlindach(DE)**

Rank Xerox (UK) Business Services
(3.10/3.6/3.3.1)

## Beschreibung

Die Erfindung betrifft ein Verfahren zum Betrieb eines Computertomographen gemäß dem ersten Teil des Patentanspruches.

Computertomographen der dritten Generation besitzen eine fest mit der Röntgenstrahlenquelle verbundene und damit um das Objekt rotierende Detektorreihe. Damit können die Detektorelemente der Detektorreihe durch auf den Fokus ausgerichtete Kollimatorbleche aus stark absorbierendem Material gegen die im Objekt erzeugte Streustrahlung abgeschirmt werden. Da aber die Kollimatorbleche - bei nicht idealer Ausrichtung oder bei Schwankungen der Fokuslage - zu einem Schattenwurf auf den Detektorelementen führen, wird der dem nominellen Rastermaß der Detektorreihe entsprechende geometrische Füllgrad in der Praxis oft deutlich unterschritten.

Computertomographen der vierten Generation verwenden einen feststehenden Detektorring (oder Teilring), der das Meßfeld umgibt. Damit ist eine Kollimierung der Detektorelemente nicht möglich oder technisch so aufwendig, daß sie nicht praktikabel ist. Ferner wären mit einem solchen Kollimator wiederum die praktischen Nachteile eines reduzierten geometrischen Füllfaktors verbunden.

Es ist bekannt, daß die außerhalb der tomographischen Schicht meßbare Streustrahlung in guter Näherung mit der Streustrahlung in der Schicht korreliert.

Bei einem in der DE-A-27 17 349 beschriebenen Computertomographen gemäß dem ersten Teil des Patentanspruches ist es deshalb möglich, die Streustrahlung mit Hilfe der zweiten Detektorreihe zu erfassen und beim Bildaufbau zu berücksichtigen.

Der Erfindung liegt die Aufgabe zugrunde, einen Computertomographen gemäß dem ersten Teil des Patentanspruches so auszubilden, daß die Eichung der Detektorelemente der zweiten, die Streustrahlung erfassenden Detektorreihe in einfacher Weise möglich ist, so daß die von der Detektorreihe für das primäre Röntgenstrahlenbündel empfangene Streustrahlung genau bestimmt werden kann und somit Streustrahlungsanteile in den Meßwerten der ersten Detektorreihe mit hoher Genauigkeit korrigiert werden können.

Diese Aufgabe ist erfindungsgemäß gelöst durch den zweiten Teil des Patentanspruches.

Bei dem erfindungsgemäßen Computertomographen sind außerhalb der tomographischen Schicht in einem vorgegebenen Abstand von der Detektorreihe für das primäre Röntgenstrahlenbündel einige zusätzliche Detektorelemente angebracht, die die zweite Detektorreihe bilden und nur die im Objekt erzeugte Streustrahlung empfangen. Der gesamte Strahlenempfänger ist in Richtung der Drehachse des Drehrahmens für die Röntgenstrahlenquelle verschiebbar, so daß jede der Detektorreihen an eine Stelle, an der nur die Streustrahlung erfaßt wird, gebracht werden kann. Auf diese Weise ist eine einfache Eichung der Detektorelemente der zweiten, im Normalbetrieb nur die Streustrahlung erfassenden Detektorreihe möglich.

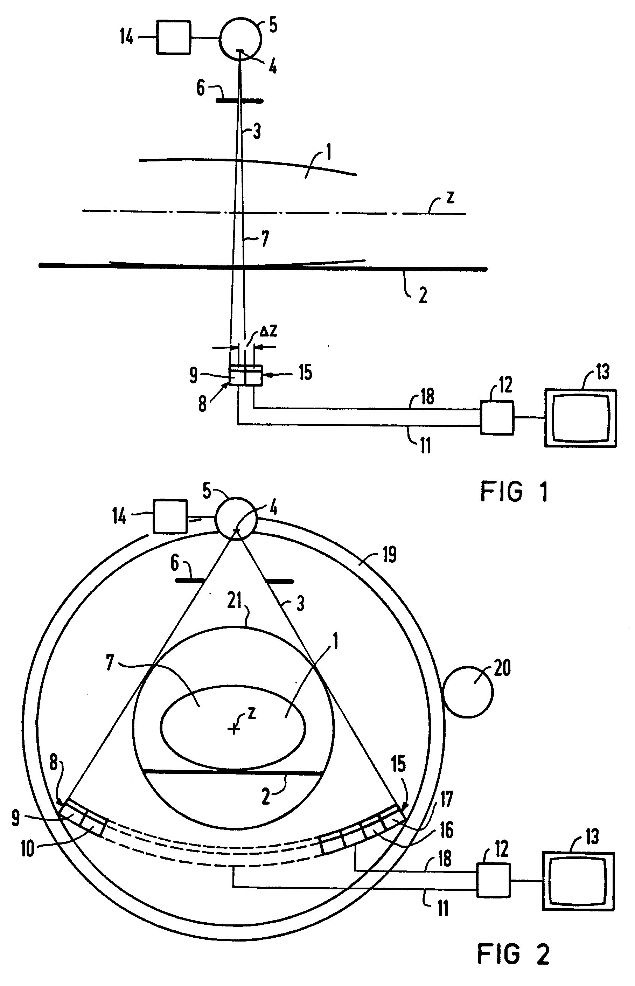

Die Erfindung ist nachfolgend anhand eines in den Fig. 1 und 2 in zwei verschiedenen Ansichten dargestellten Ausführungsbeispieles näher erläutert.

Aus den Fig. 1 und 2 geht hervor, daß ein Patient 1, der in der Fig. 2 im Querschnitt dargestellt ist, auf einer Liege 2 liegt und von einem fächerförmigen Röntgenstrahlenbündel 3 durchstrahlt wird. Das Röntgenstrahlenbündel 3 geht vom Fokus 4 einer Röntgenröhre 5 aus und ist durch eine Primärstrahlenblende 6 so eingeblendet, daß seine Querschnittsausdehnung senkrecht zur untersuchten Körperschicht 7 gleich der Schichtstärke und in der untersuchten Körperschicht 7 so groß ist, daß das Meßfeld 21, in dem der Patient 1 liegt, tangiert wird. In Strahlenrichtung gesehen nach dem Patienten 1 ist eine Detektorreihe 8 für die primäre Röntgenstrahlung angeordnet, welche aus einer Anzahl von in einer Reihe angeordneter Detektorelemente 9, 10 usw. besteht. Die Anzahl der Detektorelemente 9, 10 usw. ist der gewünschten Bildauflösung entsprechend gewählt und kann z.B. 512 betragen. Zur Abtastung des Patienten 1, und zwar der Schicht 7, wird die Meßanordnung 5, 8, 15 um 360° um den Patienten 1 gedreht. Die dabei von den Detektorelementen 9, 10 usw. gelieferten Ausgangssignale werden über eine Leitung 11 einem Computer 12 zugeführt, der daraus unter Berücksichtigung der nachfolgend beschriebenen Streustrahlungskorrektur ein Bild der durchstrahlten Körperschicht berechnet. Zur Wiedergabe dieses Bildes ist der Computer 12 an einem Sichtgerät 13 angeschlossen. Die Röntgenröhre 5 ist an einem Röntgengenerator 14 angeschlossen, welcher die erforderliche Hochspannung liefert.

Zur Berücksichtigung der Streustrahlungsintensität ist parallel zur Detektorreihe 8 und neben dieser eine Detektorreihe 15 angeordnet, welche sich während der Messung ebenfalls mit um den Patienten 1 dreht und aus einer Reihe von Detektorelementen besteht, von denen in der Fig. 2 die Detektorelemente 16 und 17 sichtbar sind. Die Detektorreihe 15 erfaßt nicht die Primärstrahlung 7, sondern nur die Streustrahlung, die praktisch gleich der Streustrahlung ist, die auf die für die eigentliche Messung benutzte Detektorreihe 8 einwirkt. Diese Messung erfolgt nach Durchführung des nachfolgend beschriebenen Eichverfahrens. Abmessungen, Lage und Gesamtzahl der Detektorelemente in der Detektorreihe 15 können verschieden gegenüber der Detektorreihe 8 sein. Die Ausgangssignale der Detektorreihe 15 sind über eine Leitung 18 ebenfalls dem Computer 12 zugeführt. Der Computer 12 enthält eine Schaltungsanordnung zur Korrektur der Ausgangssignale der Detektorreihe 8 durch die Ausgangssignale der Detektorreihe 15 in dem Sinne, daß der von der Streustrahlung herrührende Teil der Ausgangssignale der Detektorreihe 8 eliminiert wird.

Bei dem Beispiel gemäß den Fig. 1 und 2 braucht die Detektorreihe 15 nicht dieselbe Anzahl von Detektorelementen wie die Detektorreihe 8 aufzuweisen. Für eine Korrektur der Ausgangssignale der Detektorreihe 8 genügt es, wenn die Anzahl der Detektorelemente der Detektorreihe 15 wesentlich geringer als die Anzahl der Detektorelemente der Detektorreihe 8 ist.

Im Computer 12 werden die Streustrahlensignale von den Meßsignalen abgezogen.

Aus der Fig. 1 geht hervor, daß die beiden Detektorreihen 8, 15 um den Abstand $\Delta z$ in Richtung der z-Achse, d.h. der Drehachse eines Drehrahmens 19 für die Röntgenröhre 5 und die Detektorreihen 8, 15 verschiebbar sind. Die Komponenten 5, 8, 15 werden von einem Antrieb 20 gedreht. Aufgrund der Verschiebbarkeit der Detektorreihen 8, 15 ist es möglich, jede Detektorreihe 8, 15 wahlweise in das primäre Röntgenstrahlenbündel 3 oder in eine Stellung zu verstellen, in der nur die Streustrahlung erfaßt wird.

Die Eichung der Detektorelemente 16, 17 usw. der Detektorreihe 15 kann damit mit einem normierten Streukörper, z.B. einer homogenen Kreisscheibe, folgendermaßen durchgeführt werden:

In einem ersten Schritt werden die Detektorelemente 9, 10 der Detektorreihe 8 wie üblich ohne Streukörper mit Primärstrahlung relativ zueinander geeicht.

In einem zweiten Schritt wird der normierte Streukörper in den Strahlengang gebracht, und die Detektorreihe 8 axial um die Strecke $\Delta z$ verschoben, so daß sie die Streustrahlung empfängt, die später an diesem Ort von den Detektorelementen 16, 17 usw. gemessen wird.

Die so gewonnenen Meßwerte seien mit $M_i$ (i = 1, ..., $N_M$) bezeichnet, wobei $N_M$ die Anzahl der Detektorelemente 16, 17 usw. bezeichnet. Die Detektorreihe 8 selbst kann natürlich um ein Vielfaches mehr an Detektorelementen aufweisen. Es werden nur die $N_M$-Meßwerte von Detektorelementen herausgegriffen, die im Strahlenfächer, also in azimutaler Richtung, den Detektorelementen der Detektorreihe 15 gegenüberstehen.

In einem letzten Schritt wird die gesamte Detektoranordnung wieder in die axiale Ausgangsstellung gebracht und die Streustrahlung des Streukörpers jetzt mit den Detektorelementen 16, 17 usw. gemessen. Die Meßwerte seien mit $\widetilde{M_i}$ (i = 1, ...,

$N_M$) bezeichnet.

Damit sind schließlich die Eichfaktoren

$$E_i = M_i / \widetilde{M_i}$$

bekannt, mit denen die Meßwerte der Detektorelemente 16, 17 usw. zu multiplizieren sind, um die von der Detektorreihe 8 empfangene Streustrahlung zu erhalten. Damit kann die Streustrahlung in der tomographischen Schicht aus Meßwerten außerhalb der Schicht genau bestimmt werden. Durch Subtraktion der so bestimmten Streustrahlungsintensitäten von den Meßwerten der Detektorreihe 8 kann die ungestörte Primärstrahlung so genau bestimmt werden, daß die mit den üblichen Verfahren rekonstruierten Schichtbilder frei von Streustrahlungsartefakten sind.

Dieses Eichverfahren muß natürlich nicht bei jedem Objektwechsel durchgeführt werden. Diese Eichfaktoren sind als Tabelle in einem Speicher abgelegt, der dann bei erneuten Aufnahmen ausgelesen wird, womit die Korrektur mit dem beschriebenen Verfahren durchführbar ist.

**Patentansprüche**

1. Verfahren zum Betrieb eines Computertomographen zur Herstellung von Transversalschichtbildern eines Aufnahmeobjektes (1) mit einer Strahlenmeßanordnung, die eine Röntgenstrahlenquelle (5), welche ein das Aufnahmeobjekt (1) durchdringendes, fächerförmiges Röntgenstrahlenbündel (3) erzeugt, dessen Querschnittsausdehnung senkrecht zur Schichtebene gleich der Schichtstärke ist und in der Schichtebene ein Meßfeld (21) durchsetzt, sowie einen Strahlenempfänger enthält, der die Strahlungsintensität hinter dem Objekt (1) ermittelt, mit einer Drehvorrichtung (19, 20) für die Röntgenstrahlenquelle (5) und mit einem Rechner (12) für die Transformation der vom Strahlenempfänger gelieferten Signale in ein Schichtbild, bei dem der Strahlenempfänger aus zwei parallel zueinander und nebeneinander angeordneten Detektorreihen (8, 15) besteht und so angeordnet ist, daß nur eine Detektorreihe (8) vom primären Röntgenstrahlenbündel (1) getroffen wird, während die zweite Detektorreihe (15) nur die Streustrahlung erfaßt und im Rechner (12) eine Schaltungsanordnung zur Korrektur der Ausgangssignale der vom primären Röntgenstrahlenbündel (1) getroffenen Detektorreihe (8) durch die Ausgangssignale der zweiten Detektorreihe (15) vorhanden ist, wobei der Strahlenempfänger in Richtung der Drehachse (z) verstellbar gelagert ist, **gekennzeichnet durch** folgende aufeinanderfolgende Verfahrensschritte:

Es werden die Detektorelemente der im Normalbetrieb die primäre Röntgenstrahlung erfassenden Detektorreihe (8) mit Primärstrahlung geeicht.

Es wird ein normierter Streukörper in den Strahlengang gebracht und diese Detektorreihe (8) axial verschoben, so daß sie die Streustrahlung empfängt, die später von der im Normalbetrieb nur die Streustrahlung erfassenden Detektorreihe (15) gemessen wird.

Die gesamte Detektoranordnung (8, 15) wird wieder in die Ausgangsstellung gebracht und die Streustrahlung des Streukörpers mit den Detektorelementen (16, 17) der im Normalbetrieb die Streustrahlung erfassenden Detektorreihe (15) gemessen.

Durch Division der Meßwerte ($M_i$, $\widetilde{M_i}$) bei den letzten beiden Verfahrensschritten werden Eichfaktoren ($E_i$) bestimmt, mit denen die empfangenen Streustrahlungsintensitäten erhalten werden. Die Streustrahlungsintensitäten werden zur Bestimmung der Primärstrahlung von den Meßwerten der Detektorreihe (8) subtrahiert.

## Claims

1. Method for the operation of a computer tomography apparatus for the manufacture of diagonal layer images of a target object (1) with a beam-measuring arrangement, which contains an X-ray source (5) which generates a fan-shaped X-ray beam (3) penetrating the target object (1), the cross-sectional extent of which X-ray beam perpendicular to the layer plane is equal to the layer thickness and in the layer plane crosses a measuring field (21), and contains a radiation receiver which determines the radiation intensity behind the object (1), with a rotating device (19, 20) for the X-ray source (5) and with a computer (12) for the transformation of the signals supplied by the radiation receiver into a layer image, where the radiation receiver consists of two rows of detectors (8, 15) arranged parallel to one another and next to one another and is arranged in such a way that only one row of detectors (8) is struck by the primary X-ray beam (1), while the second row of detectors (15) only detects the scattered radiation and in the computer (12) a switching arrangement for the correction of the output signals of the row of detectors (8) struck by the primary X-ray beam (1) by the output signals of the second row of detectors (15) is present, whereby the radiation receiver is mounted so that it can be adjusted in the direction of the axis of rotation (z), characterized by the following successive procedural steps:

the detector elements of the row of detectors (8) detecting in normal operation the primary X-radiation are calibrated with primary radiation;

a standardized scatter body is brought into the path of rays and this row of detectors (8) is axially displaced so that it receives the scattered radiation which is later measured by the row of detectors (15) which only detects the scattered radiation in normal operation;

the entire detector arrangement (8, 15) is brought into the starting position again and the scattered radiation of the scatter body is measured by the detector elements (16, 17) of the row of detectors (15) detecting the scattered radiation in normal operation;

through division of the measured values ($M_i$, $M_i$) with the last two procedural steps, calibration factors ($E_i$) are determined, with which the received scattered-radiation intensities are obtained. The scattered-radiation intensities are subtracted from the measured values of the row of detectors (8) for the determination of the primary radiation.

## Revendications

1. Procédé d'utilisation d'un tomodensitomètre pour former des tomographies transversales d'un objet de prise de vues (1), comportant un dispositif de mesure du rayonnement, qui comporte une source de rayonnement X (5), qui produit un faisceau de rayons X en forme d'éventail (3), qui traverse l'objet de prise de vues (1) et dont l'étendue en coupe transversale perpendiculairement au plan de la couche est égale à l'épaisseur de la couche et traverse, dans le plan de la couche, une zone de mesure (21), ainsi qu'un récepteur de rayonnement, qui détermine l'intensité du rayonnement en arrière de l'objet (1), et comportant un dispositif rotatif (19,20) pour la source de rayons X (5), et un calculateur ou oridnateur (12) pour transformer des signaux délivrés par le récepteur de rayonnement en une tomographie, selon lequel le récepteur de rayonnement est constitué par deux rangées de détecteurs (8,15), parallèles entre elles et côte-à-côte, et disposées de telle sorte que seule une rangée de détecteurs (8) est atteinte par le faisceau primaire de rayons X (1), tandis que la seconde rangée de détecteurs (15) ne détecte que le rayonnement diffus, et dans le calculateur (12) est disposé un montage servant à corriger les signaux de sortie de la rangée de détecteurs (8), atteinte par le faisceau primaire de rayons X (1), au moyen des signaux de sortie de la seconde rangée de détecteurs (15), le récepteur de rayonnement étant monté de manière à être réglable dans la direction de l'axe de rotation (z), caractérisé par les phases opératoires successives suivantes :

on étalonne, avec un rayonnement primaire, les éléments détecteurs de la rangée de détecteurs (8) qui détecte, lors du fonctionnement normal, le rayonnement X primaire;

on place un corps diffusant normalisé dans le trajet du rayonnement et on déplace axialement cette rangée de détecteurs (8) de manière qu'elle reçoive le rayonnement diffus qui est mesuré ultérieurement par la rangée de détecteurs (15), qui détecte uniquement le rayonnement diffus pendant le fonctionnement normal;

on place à nouveau l'ensemble du dispositif de détecteurs (8 15) dans la position initiale et on mesure le rayonnement diffus du corps diffusant au moyen des éléments détecteurs (16,17) de la rangée de détecteurs (15) qui détectent le rayonnement diffus, pendant le fonctionnement normal; et

en divisant les valeurs de mesure ($M_i$, $M_i$) obtenues lors des deux dernières étapes opératoires, on détermine des facteurs d'étalonnage ($E_i$), à l'aide desquels on obtient les intensités reçues du rayonnement diffus, et on soustrait les intensités du rayonnement diffus, des valeurs de mesure de la rangée de détecteurs (8), pour la détermination du rayonnement primaire.

FIG 1

FIG 2